# EUROPEAN PATENT SPECIFICATION

(11) **EP 2 298 203 B1**
(45) Date of publication and mention of the grant of the patent: **08.03.2017**
(21) Application number: 10180965.5
(22) Date of filing: 24.01.2007
(51) Int. Cl.: A61B 18/12, A61B 5/053, A61B 18/14, A61B 18/00

(54) **System for terminating treatment in impedance feedback algorithm**
System zur Beendigung einer elektrochirurgischen Behandlung mittels eines Impedanzrückkoppelungsalgorithmus
Dispositif permettant l'arrêt d'une procédure électro-chirurgicale à l'aide d'un algorithme de rétroaction contrôlée en impédance

(30) Priority: 24.01.2006 US 761443 P; 24.04.2006 US 409602
(43) Date of publication of application: 23.03.2011
(62) Divisional of application: 07001485.7
(73) Proprietor: Covidien AG, 8212 Neuhausen am Rheinfall (CH)
(72) Inventor: Wham, Robert H., Boulder CO 80305 (US); Weinberg, Craig, Denver CO 80211 (US)
(74) Representative: Soames, Candida Jane

(56) References cited:
- EP-A1- 0 694 291
- US-A- 6 033 399
- US-A1- 2003 158 551
- US-A1- 2005 101 948
- US-A1- 2005 101 951

## Description

### BACKGROUND

### Technical Field

The present disclosure relates to an electrosurgical system and method for performing electrosurgical procedures. More particularly, the present disclosure relates to determining when a particular tissue treatment process is complete based on sensed tissue properties and other predefined values.

### Background of Related Art

Energy based tissue treatment is well known in the art. Various types of energy (e.g., electrical, ultrasonic, microwave, cryo, heat, laser, etc.) are applied to tissue to achieve a desired result. Electrosurgery involves application of high radio frequency electrical current to a surgical site to cut, ablate, coagulate, seal or otherwise tissue. In monopolar electrosurgery, a source or active electrode delivers radio frequency energy from the electrosurgical generator to the tissue and a return electrode carries the current back to the generator. In monopolar electrosurgery, the source electrode is typically part of the surgical instrument held by the surgeon and applied to the tissue to be treated. A patient return electrode is placed remotely from the active electrode to carry the current back to the generator.

In bipolar electrosurgery, one of the electrodes of the hand-held instrument functions as the active electrode and the other as the return electrode. The return electrode is placed in close proximity to the active electrode such that an electrical circuit is formed between the two electrodes (e.g., electrosurgical forceps). In this manner, the applied electrical current is limited to the body tissue positioned between the electrodes. When the electrodes are sufficiently separated from one another, the electrical circuit is open and thus inadvertent contact with body tissue with either of the separated electrodes does not cause current to flow.

It is known in the art that sensed tissue feedback may be used to control delivery of electrosurgical energy.

US 2005/0101948 discloses in figure 40B a graph showing a change of impedance over time of living tissue which occurs as a result of the delivery of high frequency output power. US 6,033,399 and US 2005/0101951 disclose electrosurgical control systems.

### SUMMARY

The present disclosure relates to system and method for determining completion of electrosurgical treatment. The system includes an electrosurgical generator having a microprocessor and sensor circuitry. Sensor circuitry continually monitors tissue impedance and measures offset impedance. The microprocessor compares tissue impedance to a threshold impedance value which is defined as a function of the offset impedance and a hard-coded ending impedance value. If the tissue impedance is at or above the threshold impedance value the treatment is complete and the system adjusts the output of the generator.

According to one aspect of the present disclosure an electrosurgical system is disclosed. The system includes an electrosurgical generator adapted to supply energy at an output level to tissue. The electrosurgical generator includes a microprocessor adapted to generate a desired impedance trajectory having at least one slope. The target impedance trajectory includes one or more target impedance values. The microprocessor is also adapted to drive tissue impedance along the target impedance trajectory by adjusting the output level to substantially match tissue impedance to a corresponding target impedance value. The microprocessor is further adapted to compare tissue impedance to a threshold impedance value and adjust output of the electrosurgical generator when the tissue impedance is equal to or greater than the threshold impedance. The system also includes an electrosurgical instrument including at least one active electrode adapted to apply electrosurgical energy to tissue.

According to a further aspect of the present disclosure an electrosurgical generator is disclosed. The electrosurgical generator includes an RF output stage adapted to supply electrosurgical energy to tissue. The electrosurgical generator also includes a microprocessor adapted to generate a desired impedance trajectory having at least one slope. The target impedance trajectory includes one or more target impedance values. The microprocessor is also adapted to drive tissue impedance along the target impedance trajectory by adjusting the output level to substantially match tissue impedance to a corresponding target impedance value. The microprocessor is further adapted to compare tissue impedance to a threshold impedance value and adjust output of the electrosurgical generator when the tissue impedance is equal to or greater than the threshold impedance.

Further features of the system and generator of the present invention are defined in the claims.

### BRIEF DESCRIPTION OF THE DRAWINGS

Various embodiments of the present disclosure are described herein with reference to the drawings wherein:
Fig. 1 is a schematic block diagram of an electrosurgical system according to the present disclosure;
Fig. 2 is a schematic block diagram of a generator according to the present disclosure; and
Fig. 3 is a flow diagram illustrating a method according to the present disclosure.

### DETAILED DESCRIPTION

Particular embodiments of the present disclosure will be described hereinbelow with reference to the accompanying drawings. In the following description, well-known functions or constructions are not described in detail to avoid obscuring the present disclosure in unnecessary detail. Those skilled in the art will understand that the present disclosure may be adapted for use with either an endoscopic instrument or an open instrument.

It is envisioned the method may be extended to other tissue effects and energy-based modalities including, but not limited to ultrasonic, laser, microwave, and cryo tissue treatments. It is also envisioned that the disclosed methods are based on impedance measurement and monitoring but other tissue and energy properties may be used to determine state of the tissue, such as temperature, current, voltage, power, energy, phase of voltage and current. It is further envisioned that the method may be carried out using a feedback system incorporated into an electrosurgical system or may be a stand-alone modular embodiment (e.g., removable modular circuit configured to be electrically coupled to various components, such as a generator, of the electrosurgical system).

The present disclosure relates to a method for controlling energy delivery to tissue based on tissue feedback. If electrosurgical energy is being used to treat the tissue, the tissue characteristic being measured and used as feedback is typically impedance and the interrogatory signal is electrical in nature. If other energy is being used to treat tissue then interrogatory signals and the tissue properties being sensed vary accordingly. For instance the interrogation signal may be achieved thermally, audibly, optically, ultrasonically, etc. and the initial tissue characteristic may then correspondingly be temperature, density, opaqueness, etc. The method according to the present disclosure is discussed using electrosurgical energy and corresponding tissue properties (e.g., impedance). Those skilled in the art will appreciate that the method may be adopted using other energy applications.

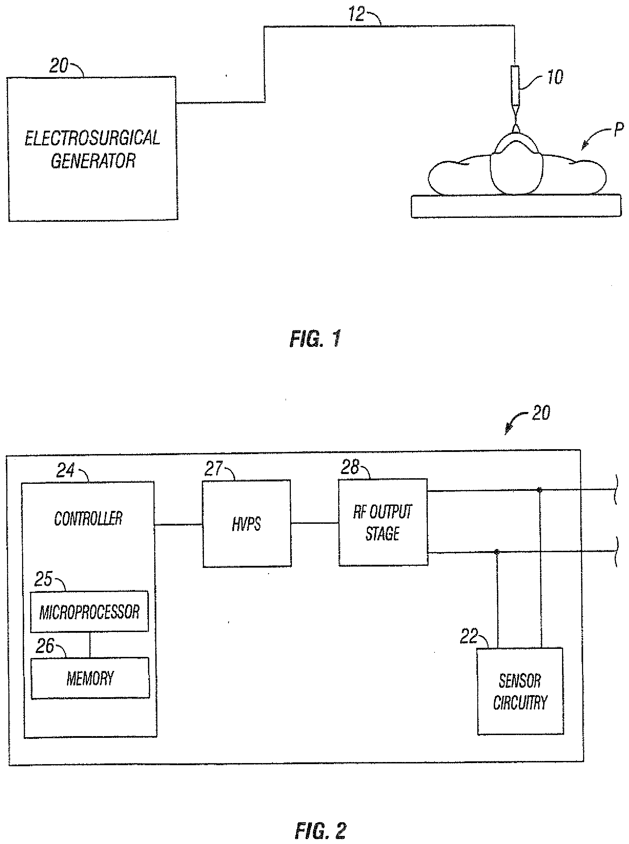

Fig. 1 is a schematic illustration of an electrosurgical system according to the present disclosure. The system includes an electrosurgical instrument 10 having one or more electrodes for treating tissue of a patient P. The instrument 10 may be either a monopolar type including one or more active electrodes (e.g., electrosurgical cutting probe, ablation electrode(s), etc.) or a bipolar type including one or more active and return electrodes (e.g., electrosurgical sealing forceps). Electrosurgical RF energy is supplied to the instrument 10 by a generator 20 via a supply line 12, which is operably connected to an active output terminal, allowing the instrument 10 to coagulate, seal, ablate and/or otherwise treat tissue.

If the instrument 10 is a monopolar type instrument then energy may be returned to the generator 20 through a return electrode (not explicitly shown) which may be disposed on the patient's body. The system may also include a plurality of return electrodes which are arranged to minimize the chances of damaged tissue by maximizing the overall contact area with the patient P. In addition, the generator 20 and the monopolar return electrode may be configured for monitoring so called "tissue-to-patient" contact to insure that sufficient contact exists therebetween to further minimize chances of tissue damage.

If the instrument 10 is a bipolar type instrument, the return electrode is disposed in proximity to the active electrode (e.g., on opposing jaws of a bipolar forceps). It is also envisioned that the generator 20 may include a plurality of supply and return terminals and a corresponding number of electrode leads.

The generator 20 includes input controls (e.g., buttons, activators, switches, touch screen, etc.) for controlling the generator 20. In addition, the generator 20 may include one or more display screens for providing the surgeon with a variety of output information (e.g., intensity settings, treatment complete indicators, etc.). The controls allow the surgeon to adjust power of the RF energy, waveform, and other parameters to achieve the desired waveform suitable for a particular task (e.g., coagulating, tissue sealing, intensity setting, etc.). It is also envisioned that the instrument 10 may include a plurality of input controls which may be redundant with certain input controls of the generator 20. Placing the input controls at the instrument 10 allows for easier and faster modification of RF energy parameters during the surgical procedure without requiring interaction with the generator 20.

Fig. 2 shows a schematic block diagram of the generator 20 having a controller 24, a high voltage DC power supply 27 ("HVPS") and an RF output phase 28. The HVPS 27 provides high voltage DC power to an RF output phase 28 which then converts high voltage DC power into RF energy and delivers the high frequency RF energy to the active electrode 24. In particular, the RF output phase 28 generates sinusoidal waveforms of high frequency RF energy. The RF output phase 28 is configured to generate a plurality of waveforms having various duty cycles, peak voltages, crest factors, and other parameters. Certain types of waveforms are suitable for specific electrosurgical modes. For instance, the RF output phase 28 generates a 100% duty cycle sinusoidal waveform in cut mode, which is best suited for dissecting tissue and a 25% duty cycle waveform in coagulation mode, which is best used for cauterizing tissue to stop bleeding.

The controller 24 includes a microprocessor 25 operably connected to a memory 26 which may be volatile type memory (e.g., RAM) and/or non-volatile type memory (e.g., flash media, disk media, etc.). The microprocessor 25 includes an output port which is operably connected to the HVPS 27 and/or RF output phase 28 allowing the microprocessor 25 to control the output of the generator 20 according to either open and/or closed control loop schemes.

A closed loop control scheme or feedback control loop is provided that includes sensor circuitry 22 having one or more sensors for measuring a variety of tissue and energy properties (e.g., tissue impedance, tissue temperature, output current and/or voltage, etc.). The sensor circuitry 22 provides feedback to the controller 24. Such sensors are within the purview of those skilled in the art. The controller 24 then signals the HVPS 27 and/or RF output phase 28 which then adjust DC and/or RF power supply, respectively. The controller 24 also receives input signals from the input controls of the generator 20 or the instrument 10. The controller 24 utilizes the input signals to adjust power outputted by the generator 20 and/or performs other control functions thereon.

In particular, sensor circuitry 22 is adapted to measure tissue impedance. This is accomplished by measuring voltage and current signals and calculating corresponding impedance values as a function thereof at the sensor circuitry 22 and/or at the microprocessor 25. Power and other energy properties may also be calculated based on collected voltage and current signals. The sensed impedance measurements are used as feedback by the generator 20.

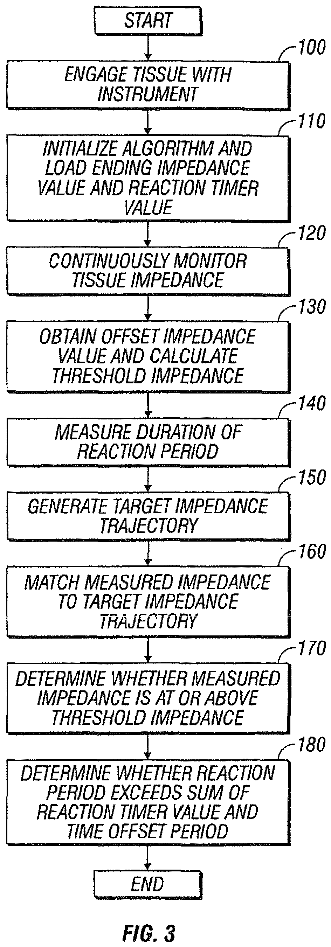

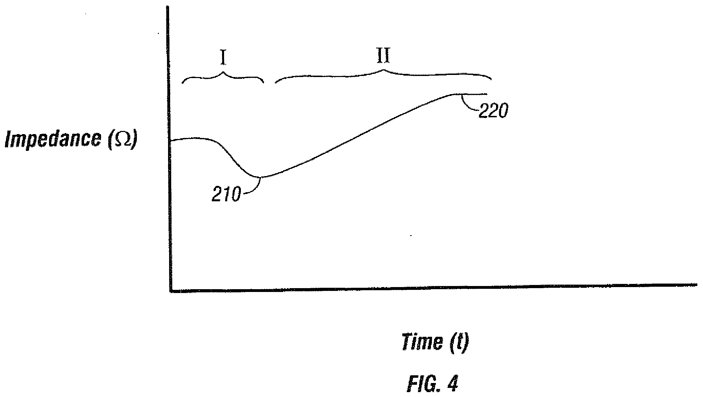

The method of sealing tissue according to the present disclosure is discussed below with reference to Fig. 3 and Fig. 4. The method may be embodied in a software-based tissue treatment algorithm which is stored in memory 26 and is executed by microprocessor 25. Fig. 4 shows an impedance over time graph illustrating various phases which tissue undergoes during particular application of energy thereto. The decrease in tissue impedance as energy is applied occurs when tissue is being fused (e.g., vessel sealing), ablated, or desiccated. It is generally known that at the onset of electrical energy (i.e., during tissue fusion, ablation, or desiccation) tissue heating results in a decreasing impedance toward a minimum value that is below the initial sensed impedance. Tissue impedance begins to rise almost immediately when tissue is being coagulated.

During phase I which is a pre-heating or early desiccation stage, the level of energy supplied to the tissue is sufficiently low and impedance of the tissue starts at an initial impedance value. As more energy is applied to the tissue, temperature therein rises and tissue impedance decreases. At a later point in time, tissue impedance reaches a minimum impedance value 210 which corresponds to tissue temperature of approximately 100° C, a boiling temperature for intra- and extra-cellular fluid boiling temperature.

Phase II is a vaporization phase or a late desiccation phase, during which tissue has achieved a phase transition from a moist, conductive to a dry, non-conductive properties. In particular, as the majority of the intra- and extra-cellular fluids begin to rapidly boil during the end of phase I, impedance begins to rise above the minimum impedance value 210. As sufficient energy is continually applied to the tissue during phase II, temperature rises beyond the boiling point coinciding with minimum impedance value 210. As temperature continues to rise, tissue undergoes a phase change from moist state to a solid state and eventually a dried-out state. As further energy is applied, tissue is completely desiccated and eventually vaporized, producing steam, tissue vapors and charring. Those skilled in the art will appreciate that the impedance changes illustrated in Fig. 4 are illustrative of an exemplary electrosurgical procedure and that the present disclosure may be utilized with respect to electrosurgical procedures having different impedance curves and/or trajectories.

Application of electrosurgical energy is controlled via an impedance feedback algorithm which controls output of the generator 20 as a function of the measured impedance signal. The impedance feedback algorithm is stored within the memory 26 and is executed by the microprocessor 26. The tissue treatment algorithm drives measured tissue impedance along a predefined target impedance trajectories (e.g., downward in phase I, upward in phase II, etc.). This is accomplished by adjusting output of the generator 20 to match measured impedance values to corresponding target impedance values. More specifically, the tissue treatment algorithm identifies when tissue has been adequately treated for desiccation, coagulation, fusion and/or sealing to halt and/or shut-off energy application.

In step 100, the instrument 10 engages the tissue and the generator 20 is activated (e.g., by pressing of a foot pedal or handswitch). In step 110, the tissue treatment algorithm is initialized and a configuration file is loaded. The configuration file includes a variety of predefined values which control the tissue treatment algorithm. In particular, an ending impedance value 220 and a reaction timer value are loaded. The ending impedance value in conjunction with an offset impedance value are used to calculate a threshold impedance value which denotes completion of treatment. In particular, application of electrosurgical energy to tissue continues until tissue impedance is at or above the threshold impedance the threshold impedance is determined by adding the ending impedance value and the offset impedance value. The ending impedance value may range from about 10 ohms to about 1000 ohms above the lowest measured impedance reached.

The termination condition may also include applying electrosurgical energy for a predetermined period of time, i.e., reaction time, which is embodied by a reaction timer value. This ensures that the treatment process does not over cook tissue. The ending impedance value 220 and the reaction timer are hard-coded and are selected automatically based on tissue type, the instrument being used and the settings selected by user. The ending impedance value 220 may be loaded at anytime during tissue treatment. Further, the ending impedance value 220 and the reaction timer may also be entered by the user.

In step 120, the generator 20 supplies electrosurgical energy to the tissue through the instrument 10. During application of energy to the tissue, impedance is continually monitored by the sensor circuitry 22. In particular, voltage and current signals are monitored and corresponding impedance values are calculated at the sensor circuitry 22 and/or at the microprocessor 25. Power and other energy properties may also be calculated based on collected voltage and current signals. The microprocessor 25 stores the collected voltage, current, and impedance within the memory 26.

In step 130, an offset impedance value is obtained. The offset impedance value is used to calculate a threshold impedance value that denotes completion of treatment. The threshold impedance is the sum of the ending impedance value 220 and the offset impedance value. The offset impedance value may be obtained in multiple ways depending on the electrosurgical procedure being performed. For example, the offset impedance may be tissue impedance measured at the time of maximum current being passed through tissue that is required to facilitate a desired tissue effect. Using the threshold impedance value referenced and partially defined by the offset impedance value rather than simply an absolute value, i.e., the ending impedance value, accounts for different tissue types, jaw fills and varying surgical devices.

Minimum measured impedance, i.e., the minimum impedance value 210, may also be used as the offset impedance value. This is particularly useful when tissue reacts normally in a desiccation process. As shown in Fig. 4, impedance drops from an initial value until the minimum impedance value 210 is reached. After a given time interval, the impedance rises again at the onset of desiccation as tissue reacts. The amount of time required for the reaction to take place and/or the minimum impedance value 210 can help define various treatment parameters by identifying type of tissue, jaw fill or a particular device being used since the minimum impedance value 210 is aligned with the beginning stage of desiccation. Consequently, the offset impedance value can be captured at the point in time when the impedance slope becomes positive, i.e., when the change of impedance over time (dzdt) is greater than zero or dzdt is approximately zero. Further, the offset impedance value may be calculated from a variety of different methods and utilizing a variety of different parameters such as: the starting tissue impedance, the impedance at minimum voltage, the impedance at either a positive or negative slope change of impedance, and/or a constant value specified within the programming or by the end user. The starting impedance may be captured at the outset of the application of the electrosurgical procedure via an interrogatory pulse.

In step 140, the timing of the reaction period is commenced to ensure that the reaction period does not exceed the reaction timer. Energy application continues until the threshold impedance value is reached before the expiration of the reaction timer. As discussed above, energy application varies for different types of tissues and procedures, therefore it is desirable that the reaction timer, similar to the threshold impedance, is also tailored to suit particular operational requirements. For this purpose, a time offset period is utilized. In particular, the time offset period is added to the reaction timer to extend the duration of energy application. Multiple time offset period values may be hard-coded (e.g., in a look-up table) so that during the procedure an appropriate value is loaded. The user may also select a desired time offset period.

The time offset period and the offset impedance values may also be obtained when measured impedance deviates from the target trajectory. Deviation from a prescribed target trajectory at any sub-segment of an energy cycle or throughout the entire cycle are tracked. When deviation is outside the prescribed threshold range for allowed deviation, which is predefined by user or hard-coded, the offset impedance and the time offset period are used in the manner described above.

In step 150, the impedance feedback algorithm calculates a target impedance trajectory based on variety of values such as: initial measured impedance, desired rate of change which is represented as a slope of the trajectory, and the like. In particular, the algorithm calculates a target impedance value at each time-step, based on a predefined desired rate of change (i.e., slope) of impedance over time (dZ/dt). The desired rate of change may be stored as a variable and be loaded during step 100 or may be selected manually or automatically based on tissue type determined by the selected instrument.

The target impedance takes the form of a target trajectory starting from a predetermined point (e.g., initial impedance value and time value corresponding to a point when tissue reaction is considered real and stable). It is envisioned that the trajectory could take a non-linear and/or quasi-linear form. The target trajectory has a positive and a negative slope as shown in Fig. 4. During coagulation and/or tissue sealing it is desirable to drive tissue impedance from a low impedance value to a high impedance value. In such circumstances the target trajectory has a linear or quasi-linear shape.

In step 160, the impedance feedback algorithm matches measured impedance to the target impedance trajectory. The impedance feedback algorithm attempts to adjust the tissue impedance to match the target impedance. While the algorithm continues to direct the RF energy to drive the tissue impedance to match the specified trajectory, the algorithm monitors the impedance to make the appropriate corrections.

In step 170, the algorithm determines whether tissue treatment is complete and the system should cease RF energy. This is determined by monitoring the actual measured impedance to determine if the actual measured impedance is at or above the predetermined threshold impedance. In step 180, the system monitors whether the amount of time to reach the threshold impedance exceeds the reaction timer plus the time offset period. If the impedance is at or above the threshold impedance and/or the sum of the reaction timer and the time offset period has expired then the algorithm is programmed to signal completion of treatment and the generator 20 is shot off or is returned to an initial state. The algorithm may also determine if the measured impedance is greater than threshold impedance for a predetermined period of time. This determination minimizes the likelihood of terminating electrosurgical energy early when the tissue is not properly or completely sealed.

Other tissue and/or energy properties may also be employed for determining termination of treatment, such as for example tissue temperature, voltage, power and current. In particular, the algorithm analyzes tissue properties and then acquires corresponding impedance values and offset times at the specified points in the tissue response or trajectory and these values or times can be stored and/or used as absolute or reference shut-off impedances and/or times in the manner discussed above.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto

## Claims

1. An electrosurgical generator (20) comprising:
an RF output stage (28) adapted to supply electrosurgical energy to tissue; and
a microprocessor (25) configured to compare tissue impedance to a threshold impedance value and adjust output of the electrosurgical generator when the tissue impedance is equal to or greater than the threshold impedance, **characterized by** the microprocessor being configured to generate a target impedance trajectory having a negative slope and a positive slope, wherein the target impedance trajectory includes a plurality of target impedance values, the microprocessor also configured to drive tissue impedance along the target impedance trajectory by adjusting the output level to substantially match tissue impedance to a corresponding target impedance value.

2. An electrosurgical generator as in claim 1, wherein the microprocessor is further configured to generate the threshold impedance value as a function of an offset impedance value and an ending impedance value.

3. An electrosurgical generator as in claim 2, wherein the offset impedance value is selected from the group consisting of an impedance value corresponding to maximum current value, a minimum impedance value and an initial impedance value.

4. An electrosurgical generator as in claim 1, wherein the microprocessor is further configured to compare duration of a reaction period to a reaction timer value and adjust output of the electrosurgical generator when the duration of the reaction period is equal to or greater than the reaction timer value.

5. An electrosurgical generator as in claim 4, wherein the microprocessor is further configured to compare duration of the reaction period to a sum of the reaction timer value and a time offset period and adjust output of the electrosurgical generator when the duration of the reaction period is equal to or greater than the sum of the reaction timer value and the time offset period.

6. An electrosurgical generator as in claim 1, wherein the target impedance trajectory is downward in a first tissue phase and upward in a second tissue phase with respect to an impedance over time graph.

7. An electrosurgical system comprising:
an electrosurgical generator (20) according to claim 1, 2, 3, 4, 5 or 6; and
an electrosurgical instrument (10) including at least one active electrode adapted to apply electrosurgical energy to tissue.

## Patentansprüche

1. Ein elektrochirurgischer Generator (20), aufweisend:
eine HF-Ausgangsstufe (28), die dazu angepasst ist, um einem Gewebe elektrochirurgische Energie zuzuführen; und
einen Mikroprozessor (25), der dazu konfiguriert ist, um eine Gewebeimpedanz mit einem Schwellenimpedanzwert zu vergleichen und eine Ausgabe von dem elektrochirurgischen Generator anzupassen, wenn die Gewebeimpedanz gleich oder größer als die Schwellenimpedanz ist, **dadurch gekennzeichnet, dass** der Mikroprozessor dazu konfiguriert ist, um eine Zielimpedanztrajektorie mit einer negativen Steigung und einer positiven Steigung zu generieren, wobei die Zielimpedanztrajektorie eine Vielzahl von Zielimpedanzwerten umfasst, wobei der Mikroprozessor auch dazu konfiguriert ist, um eine Gewebeimpedanz entlang der Zielimpedanztrajektorie zu führen, durch Anpassen des Ausgangslevels zum im Wesentlichen Entsprechen einer Gewebeimpedanz an einen korrespondierenden Gewebeimpedanzwert.

2. Ein elektrochirurgischer Generator wie in Anspruch 1, wobei der Mikroprozessor weiter dazu konfiguriert ist, um den Schwellenimpedanzwert als eine Funktion von einem Offset-Impedanzwert und einem Endimpedanzwert zu generieren.

3. Ein elektrochirurgischer Generator wie in Anspruch 2, wobei der Offset-Impedanzwert ausgewählt ist aus der Gruppe bestehend aus einem Impedanzwert entsprechend einem maximalen Stromwert, einem minimalen Impedanzwert und einem Anfangsimpedanzwert.

4. Ein elektrochirurgischer Generator wie in Anspruch 1, wobei der Mikroprozessor weiter dazu konfiguriert ist, um eine Dauer von einer Reaktionszeit mit einem Reaktionstimerwert zu vergleichen und eine Ausgabe von dem elektrochirurgischen Generator anzupassen, wenn die Dauer von der Reaktionszeit gleich oder größer als der Reaktionstimerwert ist.

5. Ein elektrochirurgischer Generator wie in Anspruch 4, wobei der Mikroprozessor weiter dazu konfiguriert ist, um eine Dauer von der Reaktionszeit mit einer Summe von dem Reaktionszeitwert und einer Zeitoffsetdauer zu vergleichen und eine Ausgabe von dem elektrochirurgischen Generator anzupassen, wenn die Dauer von der Reaktionszeit gleich oder größer als die Summe aus dem Reaktionstimerwert und der Zeitoffsetdauer ist.

6. Ein elektrochirurgischer Generator wie in Anspruch 1, wobei die Zielimpedanztrajektorie in einer ersten Gewebephase fallend ist und in einer zweiten Gewebephase ansteigend ist, hinsichtlich einer Impedanz-gegenüber-Zeit-Kurve.

7. Ein elektrochirurgisches System, aufweisend:
einen elektrochirurgischen Generator (20) nach Anspruch 1, 2, 3, 4, 5 oder 6; und
ein elektrochirurgisches Instrument (10), das wenigstens eine aktive Elektrode umfasst, die dazu angepasst ist, um elektrochirurgische Energie an Gewebe anzulegen.

## Revendications

1. Générateur électrochirurgical (20) comprenant :
un étage de sortie RF (28) conçu pour fournir de l'énergie électrochirurgicale à un tissu ; et
un microprocesseur (25) configuré pour comparer une impédance de tissu à une valeur d'impédance de seuil et pour ajuster la sortie du générateur électrochirurgical lorsque l'impédance de tissu est égale ou supérieure à l'impédance de seuil, **caractérisé par** le microprocesseur étant configuré pour produire une trajectoire d'impédance cible ayant une pente négative et une pente positive, dans lequel la trajectoire d'impédance cible inclut une pluralité de valeurs d'impédance cibles, le microprocesseur étant également configuré pour piloter l'impédance de tissu le long de la trajectoire d'impédance cible en ajustant le niveau de sortie pour faire sensiblement correspondre l'impédance de tissu à une valeur d'impédance cible correspondante.

2. Générateur électrochirurgical selon la revendication 1, dans lequel le microprocesseur est en outre configuré pour produire la valeur d'impédance de seuil comme une fonction d'une valeur d'impédance de compensation et une valeur d'impédance de terminaison.

3. Générateur électrochirurgical selon la revendication 2, dans lequel la valeur d'impédance de compensation est sélectionnée à partir du groupe constitué par une valeur d'impédance correspondant à une valeur de courant maximal, une valeur d'impédance minimale et une valeur d'impédance initiale.

4. Générateur électrochirurgical selon la revendication 1, dans lequel le microprocesseur est en outre configuré pour comparer la durée d'une période de réaction à une valeur de minuterie de réaction et pour ajuster la sortie du générateur électrochirurgical lorsque la durée de la période de réaction est égale ou supérieure à la valeur de minuterie de réaction.

5. Générateur électrochirurgical selon la revendication 4, dans lequel le microprocesseur est en outre configuré pour comparer la durée de la période de réaction à une somme de la valeur de minuterie de réaction et d'une période de décalage temporel et pour ajuster la sortie du générateur électrochirurgical lorsque la durée de la période de réaction est égale ou supérieure à la somme de la valeur de minuterie de réaction et de la période de décalage temporel.

6. Générateur électrochirurgical selon la revendication 1, dans lequel la trajectoire d'impédance cible est vers le bas dans une première phase tissulaire et vers le haut dans une seconde phase tissulaire en ce qui concerne un graphique impédance par rapport au temps.

7. Système électrochirurgical comprenant :
un générateur électrochirurgical (20) selon la revendication 1, 2, 3, 4, 5 ou 6 ; et
un instrument électrochirurgical (10) incluant au moins une électrode active conçue pour appliquer de l'énergie électrochirurgicale à un tissu.
